# EUROPEAN PATENT APPLICATION

(11) **EP 2 767 283 A1**
(43) Date of publication of application: **20.08.2014**
(21) Application number: 12840720.2
(22) Date of filing: 10.10.2012
(51) Int. Cl.: A61K 31/426, A61P 13/12, A61P 19/06, A61P 25/00, A61P 37/00, C07D 277/20, C07D 277/56

(54) **THERAPEUTIC AGENT AND PREVENTIVE AGENT FOR DEMYELINATING DISEASE**

(30) Priority: 11.10.2011 JP 2011223978
(71) Applicant: Osaka University, Suita-shi, Osaka 565-0871 (JP); Teijin Pharma Limited, Tokyo 100-0013 (JP)
(72) Inventor: NAKATSUJI, Yuji, Suita-shi Osaka 565-0871 (JP); KINOSHITA, Makoto, Suita-shi Osaka 565-0871 (JP); HONORAT, Josephe Archie, Suita-shi Osaka 565-0871 (JP); SAKODA, Saburo, Toyonaka-shi Osaka 560-0045 (JP)
(74) Representative: Carpmaels & Ransford LLP
(86) International application number: PCT/JP2012/076746
(87) International publication number: WO 2013/054940

(57) **Abstract**

The object of the present invention is to provide a novel therapeutic or preventive agent for demyelinating disease. The present invention is a therapeutic or preventive agent for demyelinating disease, containing as an active ingredient a 2-phenylthiazole compound represented by Formula (I) 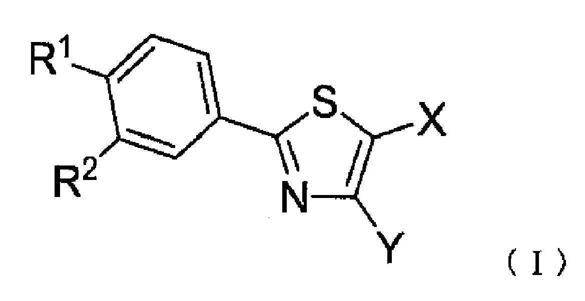 or a pharmaceutically acceptable salt thereof.

## Description

### Technical Field

The present invention relates to a therapeutic agent and a preventive agent for demyelinating disease and to a therapeutic method and a preventive method using the same.

Furthermore, the present invention relates to a therapeutic agent and a preventive agent for multiple sclerosis and to a therapeutic method and a preventive method using the same.

### Background Art

Demyelinating disease is a kind of neurological disorders that is caused by damage to myelin sheath of myelinated axon; the damage to myelin sheath reduces nerve conduction velocity, which causes various neurological symptoms. Multiple sclerosis (MS), which is a representative disease of demyelinating disease, is a kind of autoimmune disease and has multifocal demyelinating lesions generated mainly in white matter such as the optic nerve, cerebrum, cerebellum and brainstem. Visual impairment, weakness, numbness, dizziness, fatigue, shivering and dysuria are the common symptoms. Usually the disease exhibits relapse and remitting phase and gradually progresses leading to the severe disability in the end.

Although two kinds of interferon (IFN)- β-1 agents are currently available for the prophylactic treatment of MS, these preparations impose a heavy burden on patients because these preparations are injections with short dosage intervals which require multiple-dose. Furthermore, other problems are reduction of therapeutic effect caused by the appearance of neutralizing antibody and frequently occurrence of untoward effects such as occurrence of psychiatric symptoms including depression, flu-like symptoms and injection site reaction. Recently, though fingolimod hydrochloride, a novel immunosuppressive agent, was approved as an oral therapeutic agent for MS, this agent includes severe adverse effects, and it cannot necessarily be said that the present alternatives for the treatment of MS are sufficient. The development of a novel treatment is strongly required.

It is known that 2-phenylthiazole compounds such as 2-(3-cyano-4-isobutyloxyphenyl)-4-methyl-5-thiazolecarboxylic acid used in the present invention inhibit xanthine oxidase to have action which lowers uric acid and are potentially be a therapeutic agent for hyperuricemia and gout (Non-Patent Document 1), and to have action which preserves renal function and are potentially be a therapeutic agent for impaired renal function (Patent Document 1), and the like. However, it is not known that 2-phenylthiazole compounds have an effect of treating or preventing demyelinating disease and multiple sclerosis.

### Citation List

### Patent Document

Patent Document 1: International Publication No. WO2008/064015

### Non-patent Document

Non-patent Document 1: Arthritis and Rheumatism 2005; 52:916-923

### Summary of the Invention

### Problems to be Solved by the Invention

It is an object of the present invention to provide a novel therapeutic or preventive agent for demyelinating disease. Furthermore, it is an object of the present invention to provide a novel therapeutic or preventive agent for multiple sclerosis.

### Means for Solving the Problems

As a result of eager investigations into such problems, the present inventors have found that 2-phenylthiazole compounds used in the present invention have an effect of treating or preventing demyelinating disease or multiple sclerosis.

That is, the present invention is
(1) a pharmaceutical composition for treating or preventing demyelinating disease, comprising as an active ingredient a 2-phenylthiazole compound represented by Formula (I) 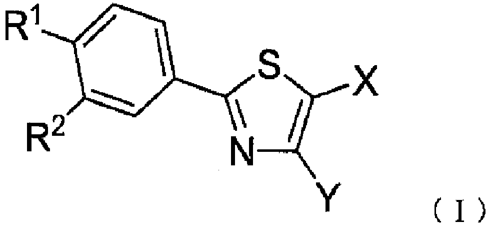 or a pharmaceutically acceptable salt thereof, wherein
   R¹ is a C₁₋₈ alkoxy group, morpholino group, 4-methylpiperazin-1-yl group or piperidino group;
   R² is a nitro group or cyano group;
   X is a carboxyl group or C₂₋₇ alkoxycarbonyl group; and
   Y is a hydrogen atom or C₁₋₆ alkyl group.
   Further, the present invention is a therapeutic or preventive agent for multiple sclerosis, containing as an active ingredient a 2-phenylthiazole compound represented by the above-mentioned Formula (I) or a pharmaceutically acceptable salt thereof;
(2) the therapeutic or preventive agent according to (1), wherein the demyelinating disease is associated with hyperuricemia or gout;
(3) the therapeutic or preventive agent according to (1), wherein the demyelinating disease is associated with impaired renal function;
(4) the therapeutic or preventive agent according to any one of (1) to (3), wherein the demyelinating disease is multiple sclerosis;
(5) the therapeutic or preventive agent according to any one of (1) to (4), wherein the 2-phenylthiazole compound represented by the above-mentioned Formula (I) is 2-(3-cyano-4-isobutyloxyphenyl)-4-methyl-5-thiazolecarboxylic acid or a pharmaceutically acceptable salt thereof.
   Furthermore, the present invention relates to
(6) a therapeutic or preventive method for demyelinating disease, including administration of an effective amount for treating or preventing demyelinating disease of the 2-phenylthiazole compound represented by the above-mentioned Formula (I) or a pharmaceutically acceptable salt thereof;
(7) the therapeutic or preventive method according to (6), wherein the demyelinating disease is associated with hyperuricemia or gout;
(8) the therapeutic or preventive method according to (6), wherein the demyelinating disease is associated with impaired renal function;
(9) the therapeutic or preventive method according to any one of (6) to (8), wherein the demyelinating disease is multiple sclerosis; and
(10) the therapeutic or preventive method according to any one of (6) to (9), wherein the 2-phenylthiazole compound represented by the above-mentioned Formula (I) is 2-(3-cyano-4-isobutyloxyphenyl)-4-methyl-5-thiazolecarboxylic acid or a pharmaceutically acceptable salt thereof.

### Advantages of the Invention

According to the present invention, use of a 2-phenylthiazole compound or a pharmaceutically acceptable salt thereof, which is used in the present invention, can treat or prevent demyelinating disease or multiple sclerosis.

### Brief Description of the Drawings

FIG. 1 shows a graph of changes in the neurologic symptom scores of Example 1.
FIG.2 shows photographs of histopathological images of the spinal cords of Example 1.

### Mode for Carrying Out the Invention

A 2-phenylthiazole compound represented by Formula (I) 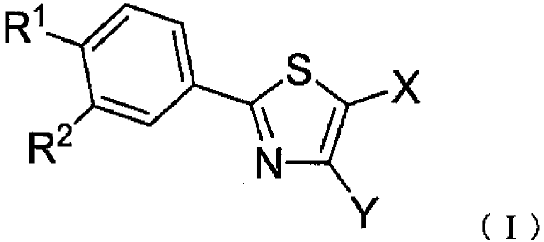 or a pharmaceutically acceptable salt thereof, which is used in the present invention, wherein
R¹ is a C₁₋₈ alkoxy group, morpholino group, 4-methylpiperazine-1-yl group or piperidino group;
R² is a nitro group or cyano group;
X is a carboxyl group or C₂₋₇ alkoxycarbonyl group; and
Y is a hydrogen atom or C₁₋₆ alkyl group,
includes, for example, 2-(3-cyano-4-isobutyloxyphenyl)-4-methyl-5-thiazolecarboxylic acid. Further, a compound represented by Formula (I) can be prepared by publicly-known methods such as a method described in International Publication No.WO92/09279.

The "C₁-C₈ alkoxy group" for R¹ in Formula (I) means a group consisting of an oxy group and a C₁-C₈ straight-chain or a branched alkyl group such as a methyl, ethyl, n-propyl, n-butyl, n-pentyl, n-hexyl, n-heptyl, n-octyl, isopropyl, isobutyl, *sec*-butyl, *ter*t-butyl, isopentyl, neopentyl, *tert*-pentyl, isohexyl, 2-methylpentyl, 1-ethylbutyl group, and the like, suitably including a methoxy, ethoxy, n-propyloxy, n-butyloxy, isopropyloxy, isobutyloxy, *sec-*butyloxy, *tert*-butyloxy, isopentyloxy and neopentyloxy group, and the like, and especially a isobutyloxy group is a preferred specific example. A C₁₋₈ alkoxy group is a preferred specific example for R¹ and especially an isobutyloxy group is preferred.

For R², a cyano group is preferred.

The "C₂-C₇ alkoxycarbonyl group" for X in Formula (I) means a group consisting of a C₁-C₆ alkoxy group in the above "C₁-C₈ alkoxy group" for R¹ and a carbonyl group, suitably including a methoxycarbonyl, ethoxycarbonyl group, and the like. A Carboxyl group is a preferred specific example for X.

The "C₁-C₆ alkyl group" for Y in Formula (I) means a C₁-C₆ straight-chain or a branched alkyl group such as a methyl, ethyl, n-propyl, n-butyl, n-pentyl, n-hexyl, isopropyl, isobutyl, *sec-*butyl, *tert*-butyl, isopentyl, neopentyl, *tert*-pentyl, isohexyl, 2-methylpentyl, 1-ethylbutyl group, and the like, and methyl, ethyl, propyl, and isopropyl group is a preferred specific example and especially a methyl group is preferred. A C₁₋₆ alkyl group is a preferred specific example for Y and especially a methyl group is preferred.

For compound represented by Formula (I), 2-(3-cyano-4-isobutyloxyphenyl)-4-methyl-5-thiazolecarboxylic acid is preferred.

A compound represented by the above-mentioned Formula (I) can be converted into a pharmaceutically acceptable salt as necessary. Examples of such a salt include a salt thereof with an inorganic acid such as hydrochloric acid, hydrobromic acid, hydriodic acid, sulfuric acid, nitric acid, phosphoric acid and carbonic acid; a salt thereof with an organic acid such as formic acid, acetic acid, propionic acid, trifluoroacetic acid, phthalic acid, oxalic acid, malonic acid, succinic acid, fumaric acid, maleic acid, lactic acid, malic acid, tartaric acid, citric acid, benzoic acid, methanesulfonic acid, ethanesulfonic acid, benzenesulfonic acid and p-toluenesulfonic acid; a salt thereof with an amino acid such as lysine, arginine, ornithine, glutamic acid and aspartic acid; a salt thereof with an alkali metal such as sodium, potassium and lithium; a salt thereof with an alkaline earth metal such as calcium and magnesium; a salt thereof with a metal such as aluminium, zinc and iron; a salt thereof with an organic base such as methylamine, ethylamine, t-octylamine, diethylamine, trimethylamine, triethylamine, ethylenediamine, piperidine, piperazine, pyridine, picoline, ethanolamine, diethanolamine, triethanolamine, cyclohexylamine, dicyclohexylamine, N-methylglucamine, tris(hydroxymethyl)aminomethane and N,N'-dibenzylethylenediamine; ammonium salt and the like.

As an active ingredient of the present invention, any dosage form such as solid preparations, semi-solid preparations and liquid preparations, and preparations for any application of oral and parenteral preparations (injections, transdermal preparations, ophthalmic solutions, suppositories, nasal agents, inhalants and the like) can be used.

A therapeutic or preventive agent for multiple sclerosis and the like, containing as an active ingredient a 2-phenylthiazole compound or a pharmaceutically acceptable salt thereof of the present invention is prepared using a carrier, excipient, and other additives which are usually used for preparation. A carrier or excipient for preparations can be in the solid or liquid state, which includes, for example, lactose, magnesium stearate, starch, talc, gelatin, agar, pectin, gum arabic, olive oil, sesame oil, cocoa butter, ethylene glycol and other usual materials. Administration can be in any forms of oral administration with tablets, pills, capsules, granules, powdered medicine, solution and the like; injections such as intravenous, intramuscular injections; and parenteral administration with suppositories, transdermal preparations and the like.

The dose of an active ingredient of the present invention is an effective amount for treating or preventing demyelinating disease, demyelinating disease associated with hyperuricemia or gout, demyelinating disease associated with impaired renal function, multiple sclerosis, multiple sclerosis associated with hyperuricemia or gout or multiple sclerosis associated with impaired renal function and can be determined depending on the condition, age or body weight of a patient, the kind of combined therapy, the frequency of treatment, the kind of desired effect, the administration method or the like. Each dose of the active ingredient for an adult is normally more or less 0.5 to 1000 mg per day; the active ingredient can be administered daily or intermittently, normally, once to three times/day or once to three times/week. A preparation is preferably prepared so as to meet such conditions.

Demyelinating disease in the present invention includes demyelinating disease associated with hyperuricemia or gout and demyelinating disease associated with impaired renal function. Further, multiple sclerosis in the present invention includes multiple sclerosis associated with hyperuricemia or gout and multiple sclerosis associated with impaired renal function.

Regardless of the kind of causal renal disease, impaired renal function of the present invention is defined as a state in which protein or albumin above the normal level is continuously excreted in urine: For example, mildly impaired renal function is defined as a state in which the urinary albumin excretion rate is 30 mg/day or more, or 20 µg/min or more, or 30 mg/g creatinine (mg/gCr, the urinary albumin-creatinine ratio) or more, and/or a state in which estimated glomerular filtration rate (eGFR) calculated from a serum creatinine value in accordance with the criterion of each country is 60 mL/min/1.73 m² or less; moderate to severe cases are defined, for example, as a state in which the urinary protein excretion rate is 0.5 g/day or more or the urinary albumin excretion rate is 300 mg/day or more, or 200 µg/min or more, or 300 mg/g creatinine (mg/gCr, the urinary albumin-creatinine ratio) or more, and/or a state in which estimated glomerular filtration rate (eGFR) calculated from a serum creatinine value in accordance with the criterion of each country is 30 mL/min/1.73m² or less. As diseases which cause these states, diabetic renal disease, chronic glomerulonephritis, nephrotic syndrome, IgA nephropathy and the like can be listed.

### Examples

### [Example 1]

### Investigation of the effect of 2-(3-cyano-4-isobutyloxyphenyl)-4-methyl-5-thiazolecarboxylic acid on animal model of MS.

In mouse experimental autoimmune encephalomyelitis (EAE) models, which are MS animal models, the effects of 2-(3-cyano-4-isobutyloxyphenyl)-4-methyl-5-thiazolecarboxylic acid (Compound A) were investigated. Proteolipid protein (PLP) and complete Freund's adjuvant were administered subcutaneously to 8-week-old female SJL/J mice for immunization to induce EAE. The animals were divided into 3 groups; tap water, 1.5 mg/kg/day of Compound A and 0.75 mg/kg/day of Compound A were administered to the first, second and third group, respectively, by administration in drinking water from Day 10 to Day 44 after the immunization day. Neurological symptoms in the extremities were monitored daily from the day of immunization to Day 44 post-immunization, and the observations were scored as clinical symptoms (Vsevolod Smolianov, et al., Alteration of T cell cytokine production in PLPp-139-151-induced EAE in SJL mice by animmunostimulatory CpG Oligonucleotide. Journal of Neuroinflammation 2011, 8:59).

Further, on Day 16 post-immunization at the time when the clinical symptoms showed the peak, histopathological examination was carried out by preparing spinal cord tissues from some of the mice.

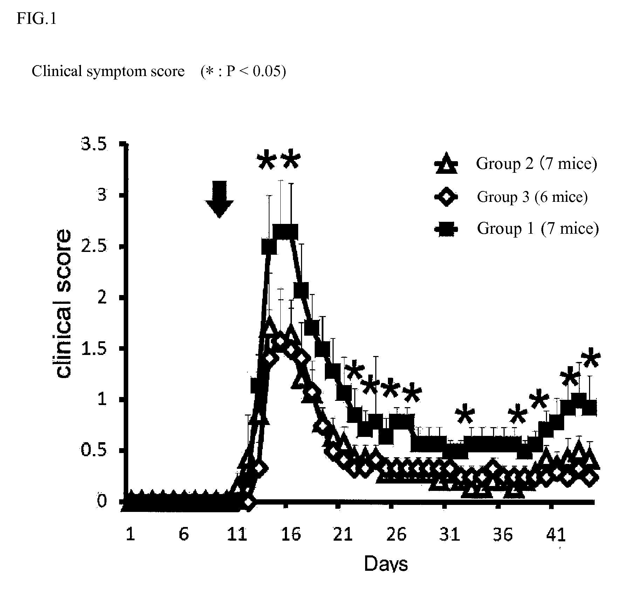

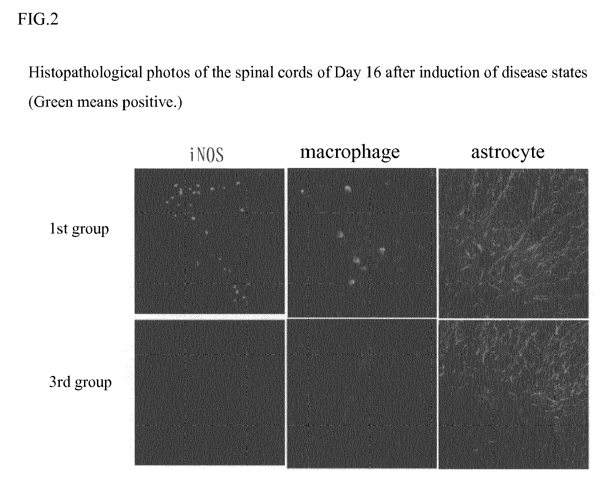

FIG. 1 shows changes in the clinical symptom scores from the day of immunization to the end of the study. Clinical symptoms of EAE were suppressed in both of Compound A-treated group 2 and group 3, in comparison with the tap water-treated group 1. In the spinal cord tissues of Day 16 post-immunization, the staining of inducible nitric oxide synthase (iNOS), macrophages and astrocytes, which were labeled with green fluorescent dye, was weak in Compound A-treated group 3 in comparison with the tap water-treated group 1 as shown in FIG. 2. Thus, it was observed that Compound A suppressed inflammation in the tissues by the reduction of iNOS-positive cells, activated macrophages, the activation of astrocytesand the like.

The evaluation has revealed that Compound A suppresses the clinical symptoms of EAE.

### Industrial Applicability

The present invention can be used for the treatment or prevention of demyelinating disease or multiple sclerosis.

## Claims

1. A therapeutic or preventive agent for demyelinating disease, comprising as an active ingredient a 2-phenylthiazole compound represented by Formula (I) 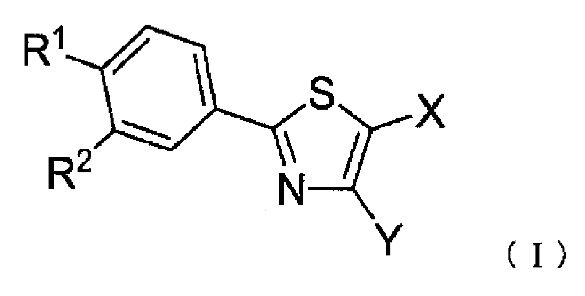 or a pharmaceutically acceptable salt thereof, wherein
R¹ is a C₁₋₈ alkoxy group, morpholino group, 4-methylpiperazin-1-yl group or piperidino group;
R² is a nitro group or cyano group;
X is a carboxyl group or C₂₋₇ alkoxycarbonyl group; and
Y is a hydrogen atom or C₁₋₆ alkyl group.

2. The therapeutic or preventive agent according to Claim 1, wherein the demyelinating disease is associated with hyperuricemia or gout;

3. The therapeutic or preventive agent according to Claim 1, wherein the demyelinating disease is associated with impaired renal function;

4. The therapeutic or preventive agent according to any one of Claims 1 to 3, wherein the demyelinating disease is multiple sclerosis;

5. The therapeutic or preventive agent according to any one of Claims 1 to 4, wherein the 2-phenylthiazole compound represented by the above-mentioned Formula (I) is 2-(3-cyano-4-isobutyloxyphenyl)-4-methyl-5-thiazolecarboxylic acid or a pharmaceutically acceptable salt thereof.
